# EUROPEAN PATENT SPECIFICATION

(11) **EP 2 764 891 B1**
(45) Date of publication and mention of the grant of the patent: **13.07.2016**
(21) Application number: 14153926.2
(22) Date of filing: 05.02.2014
(51) Int. Cl.: A61N 1/372, A61N 1/375

(54) **Implantable stimulation device, stimulation system and method for data communication**
Implantierbare Stimulationsvorrichtung, Stimulationssystem und Verfahren zur Datenkommunikation
Dispositif de stimulation implantable, système de stimulation et procédé de communication de données

(30) Priority: 07.02.2013 US 201361761707 P; 21.11.2013 US 201361906902 P
(43) Date of publication of application: 13.08.2014
(73) Proprietor: BIOTRONIK SE & Co. KG, 12359 Berlin (DE)
(72) Inventor: Baru, Marcelo, Tualatin, Oregon 97062 (US); Moulder, J. Christopher, Portland, Oregon 97202 (US)
(74) Representative: Galander, Marcus

(56) References cited:
- EP-A1- 2 508 226
- EP-A1- 2 540 340
- US-A- 5 769 877
- US-A- 5 833 710
- US-A1- 2006 122 657
- US-A1- 2010 114 248
- US-A1- 2011 190 849
- US-A1- 2012 179 218
- US-A1- 2012 259 382

## Description

Embodiments in this application generally relate to implantable medical devices and more particularly to communication techniques to transmit data from the implanted medical device to an external programmer.

Implantable stimulation devices, in particular implantable medical devices, such as implantable therapy and/or monitoring devices including pacemakers, cardioverters and defibrillators or the like, may include data communication means to transmit data from the implantable stimulation device to a (body-)external device or vice versa.

A system for data communication with an implantable stimulation device thus may include an implantable stimulation device and an external device such as a programmer.

A typical implantable stimulation device comprises a battery, a monitoring and/or therapy control unit, in some cases one or more therapy units such as stimulation units and a memory for storing control program and/or data sensed by the implantable stimulation device. If the implantable stimulation device is a pacemaker or an implantable cardioverter/defibrillator (ICD), the therapy units comprise stimulation (pacing) units for generating and delivering electric stimulation pulses to a patient's heart tissue (myocardium). Often, sensing units for sensing cardiac activity are provided. Sensing units can process electrical signals that represent electrical potentials that can be picked up via electrodes, e.g., in a heart.

In order to transmit data sensed by the implantable stimulation device to an external device or to other implanted devices, a telemetry unit may be provided. Typically the telemetry unit is configured to allow a bidirectional data communication, that is, the telemetry unit can transmit and receive data wirelessly.

Limited battery capacity of an implantable stimulation device calls for energy-efficient data communication. An implantable stimulation device with limited battery power requires a low-power communication scheme in order to program it and to download acquired data. With extremely low-power communication, more data can be transmitted more often.

The implantable stimulation device must source all of the energy required for transmitting data in all of the existing solutions. Pulses generated for data transmission use either current or voltage to create an electric field that is sensed at the receiver. Thus, the receiver passively decodes the communication and the transmitter must use sufficient energy to enable the receiver to sense the signal. This arrangement is detrimental to extremely low-power devices, such as intracardiac leadless pacemakers (iLP). The available energy for these devices is extremely limited making high-energy pulses problematic. The pulses for data transmission must also be sub-threshold because clinical complications could occur if communication caused capturing of surrounding tissue, e.g. myocardium of the heart. To reduce the possibility of capturing the heart, very short transmission windows must be used to limit communication during times when the heart is refractory to stimulation. These reduced communication times precipitate the use of high-data transmission rates that subsequently require higher clock rates for the implantable device. The higher clock rates complicate design and increase power consumption.

To avoid the problem of erroneous stimulation, it is known to encode data on the pacing pulse itself; c.f. US 2011/0208260 A1. This method ensures that the heart will be stimulated at the proper time and clinical complications will not occur. The main drawback, however, is that pacing pulse gating with return to baseline is required which substantially limits data rate and is dependant on pacing rate.

Present state-of-the-art communication schemes utilize sub-threshold pulses to galvanically transmit data to another device. When supra-threshold pulses are used, the data is encoded within the pulse itself. The information in either case is transmitted by pulses that are received and decoded. The electric field generated by the pulses is detected at the receiver, where all of the energy required for transmission is generated at the transmitter.

US 2012/0078322 teaches the use of biphasic pulses to maintain charge balancing while communicating.

EP 2508226 A1 and US 2012/0259382 A1 disclose a device and/or system for generating arbitrary scalable waveforms of a desired shape that can be used for generating a stimulation pulse for medical purposes.

US 2012/0179218 discloses an output stage for use in a therapeutic defibrillator which enables use of specialized output waveforms optimized for cardiac defibrillation. For this purpose, a pulse-width modulated switching amplifier, connected to a high voltage source capacitor and to one or more output bridges, is used.

US 2006/0122657 teaches a programmable voltage-waveform generating battery power source for implantable medical use which enables an implantable device to deliver therapeutic electrical energy with flexible control of voltage amplitude, waveform and timing. The power source includes a high-energy battery system, a waveform control system and a power amplifier.

US 8,412,352 teaches a device where the fixation mechanism is also connected to the communications module. The module uses electric pulses to communicate with another device.

US 7,945,333 teaches the combination of a programmer and an implantable device that communicate using encoded pulses. These pulses are described in the specification as modulated pacing pulses but the claim is broader. Thus if the data is encoded on pacing pulses then supra-threshold pulses are used.

Present communication schemes used or data communication by a telemetry unit involve either RF or magnetic communication. RF frequencies of ∼400 or ∼900 MHz or magnetic coupling in the 100s of kHz range require several mA of current to transmit and receive data. Such high current requirements are out of reach of devices with battery capacities of at most a few hundred mAh.

In addition, RF schemes require large antennas and magnetic coupling requires large transmit and receive coils for communication. The space available in an iLP (intracardiac leadless pacer), for instance, would not allow such large coils or antennas. iLPs are designed to be placed within a heart chamber as opposite to conventional pacemakers, where the pacemaker itself is placed outside the heart and electrode leads extend from the pacemaker into the heart.

US 6,704,602 B2 shows a medical device communication system using sub-threshold pulses for electrical communication with external devices. The medical device communication system includes an implantable medical device and external devices. The external devices can be connected to the skin of a body with a plurality of electrodes. The implantable medical device has stimulation electrodes, surface electrodes in contact with tissue of a patient, and a can including a pulse generation circuit. An electrode switching circuit is coupled to the pulse generation circuit and serves to deliver electrical stimulation pulses to the stimulation electrodes as therapy to a patient. Furthermore it serves to deliver sub-threshold pulses to the surface electrodes of the can in a predetermined pattern of modulations constituting an encoded data signal that propagates as a signal transmission through the patient tissue. The plurality of electrodes connected to the external device serve to receive the sub-threshold pulses and allow the external device to detect the encoded data signal.

US 2012/0109236 A1 presents a system for pacing a heart of a human including a leadless pacemaker in a hermetic housing with at least two electrodes and at least one external device with at least two skin electrodes. The electrodes are configured to deliver energy to stimulate a heart and to transfer information to or from the skin electrodes of the external devices. The information is preferably encoded in sub-threshold pulses delivered by the electrodes and generated by a pulse generator in the housing of the leadless pacemaker. The hermetic housing of the leadless pacemaker can further comprise a controller configured to communicate with the external devices by transferring information through the electrodes. The controller can be configured to communicate with the external devices outside of a refractory period or pacing pulse.

In view of the above, there is a need for a communication scheme that does not employ RF or magnetic coupling.

It is an object of the invention to provide an alternative implantable device and an alternative data communication method that minimizes battery drain from the stimulation device's battery.

According to one embodiment, this object is achieved by an implantable stimulation device which comprises stimulation means and data communication means and wherein the stimulation means comprise or are connected or can be connected to at least two electrodes that are configured to allow delivery of stimulation pulses and wherein the stimulation means further comprise
- a voltage source that is connected to the electrodes via at least one stimulation-pulse-switch that controls delivery of a pacing pulse
- a DC-blocking capacitor that is connected in series with the voltage source and one of the electrodes, and
- an autoshort switch that is arranged and configured to allow discharging the DC-blocking capacitor via the electrodes when the autoshort switch is closed.

The data communication means comprise data transmission control means that are connected to the autoshort switch and/or the stimulation-pulse-switch and that are configured to alternatingly open and close the autoshort switch or the stimulation-pulse-switch, respectively, during an autoshort period following the delivery of a stimulation pulse or during a stimulation pulse period, respectively, to thus modulate an autoshort pulse or a stimulation pulse, respectively.

Typically, the voltage source comprises a capacitor that is charged prior to delivery of a stimulation pulse for pacing a human heart. A blocking capacitor is provided to block delivery of DC-voltage to the tissue to be stimulated.

The present invention allows an implantable stimulation device with limited battery supply the ability to transmit increased amount of data while using reduced power. Data transmission is achieved by modulating a local electric field generated by the implantable stimulation device and read by the receiver. Continuous medium rate data transmission can be achieved while using reduced battery power.

The switching means preferably are connected to a switch control that is configured to sense an oscillatory electric field imposed on body tissue surrounding the implantable stimulation device when the implantable stimulation device is in its implanted state. Thus it is possible to synchronize switching of the autoshort switch and/or the stimulation-pulse-switch with the oscillatory electric field imposed on the body tissue surrounding or encompassing the implantable stimulation device.

To implement such synchronizing, it is preferred that the switch control comprises a phase-locked loop (PLL) and a frequency divider, wherein the phase-locked loop is configured to lock in a frequency of an oscillatory electric field imposed on body tissue surrounding the implantable stimulation device when the implantable stimulation device is in its implanted state. The frequency divider is connected to the phase-locked loop and is configured to divide a frequency signal put out by the phase-locked loop. Thus, the implantable stimulation device can generate a code that represents data to be transmitted from the implantable stimulation device to an external device, wherein the clock for such code is a fraction of the frequency of the oscillatory electric field imposed on the body tissue surrounding the implantable stimulation device. The clock frequency is preferably in a range of 0.1 to 50 kHz, such as a range of 1 to 20 kHz, more preferably in a range of 7 to 9 kHz, most preferably the clock frequency is 8 kHz.

According to a further embodiment, the switch control is connected to the at least two electrodes and is configured to sense an oscillatory electric field imposed on body tissue via the at least two electrodes. The switch control can comprise a band-pass filter, wherein the band-pass filter filters a signal fed to the phase-locked loop.

The object of the invention is further achieved by a data communication system including an implantable stimulation device as described above and an external device that comprises or can be connected to at least two cutaneous electrodes. The external device further comprises external field generating means that are configured to generate an oscillatory electric field to be transcutaneously imposed on a body via the at least two cutaneous electrodes. The external device further comprises means for sensing alterations of body impedance and/or a local electric field generated by the implantable stimulation device when the implantable stimulation device is in its implanted state.

The external device preferably comprises a lock-in amplifier, an AM demodulator for demodulating amplitude-modulated signals and an analog-to-digital converter, wherein the analog-to-digital converter is operatively connected to the AM demodulator and the lock-in amplifier, and wherein the analog-to-digital converter puts out a signal that represents a signal transmitted by the implantable stimulation device.

In a preferred embodiment the implantable device, i.e. implantable stimulation device, has a hermetically sealed housing. The hermetically sealed implantable device or implantable stimulation device with a hermetically sealed housing can be a medical therapy and/or monitoring device.

According to a further embodiment, a method of communicating data from an implantable stimulation device to an external device is provided, said method comprising:
- altering a local electric field in the body by means of the implantable stimulation device,
- by modulating an autoshort pulse and/or a stimulation pulse peak amplitude that is delivered by the implantable stimulation device,
- sensing the change of the local electric field caused by the modulation of the autoshort pulse and/or a stimulation pulse peak amplitude, respectively, by means of an external device having or being connected to at least two cutaneous electrodes.

Additionally, the method can comprise before the step of altering a local electric field the steps of:
- imposing an oscillatory electric field in body tissue encompassing an implantable stimulation device,
- sensing the imposed oscillatory electric field by means of the implantable stimulation device,

Preferably, the step of altering the local electric field by means of the implantable stimulation device is performed by means of at least two electrodes operatively connected or being part of the implantable stimulation device, and switching means operatively connected to the at least two electrodes, wherein the switching means controlled to modulate a stimulation pulse peak amplitude and/or an autoshort pulse to a code-representing data to be transmitted from the implantable stimulation device to the external device, said modulating causing a detectable change of a local electric field.
Preferably, the modulation of a stimulation pulse peak amplitude comprises amplitude changes without return to a baseline amplitude.

According to a preferred embodiment, a transceiver is utilized that imparts an electric field across the implantable stimulation device, a lock-in amplifier to detect changes in the local field around the implantable device, and an implantable device that uses charge to alter the local electric field.

During an autoshort period following delivery of a stimulation pulse, the implantable device uses the residual charge from the pacing pulse and modulates the balancing of such charge to effect communication. In this case the residual charges form the local electric field that is altered to effect communication. If communication is required during the delivery of a stimulation pulse, fast modulation of the stimulation-pulse peak amplitude without return to baseline effects communication instead. In this case the stimulation pulse forms the local electric field that is altered to effect communication.

Amplifying the local impedance or electric field change around the implantable stimulation device according to the invention increases the signal-to-noise ratio and makes communication more reliable. Also, allowing communication while pacing increases the allowable communication time for data communication.

The above and other aspects, features and advantages of the present invention will be more apparent from the following more particular description thereof, presented in conjunction with the following drawings, wherein:
- Figure 1:: is a representation of a communication system, comprising an implantable stimulation device in its implanted state and an external device;
- Figure 2:: is a more abstract representation of the system depicted in Fig. 1;
- Figure 3:: is a stimulation pulse showing modulation of the autoshort period;
- Figure 4:: is a schematic representation of a pacing circuit for data transmission during the autoshort period;
- Figure 5:: is a stimulation pulse showing modulation of the peak amplitude without return to baseline; and
- Figure 6:: is a schematic representation of a pacing circuit for data transmission during a stimulation pulse.
- Figure 7:: is a representation of an embodiment of an implantable stimulation device showing elements that allow synchronization of the switching with an imposed oscillatory electric field;
- Figure 8:: is a more detailed representation of an external device showing those elements that are provided to implement the invention;

The following description is of the best mode presently contemplated for carrying out the invention. This description is not to be taken in a limiting sense, but is made merely for the purpose of describing the general principles of the invention. The scope of the invention should be determined with reference to the claims.

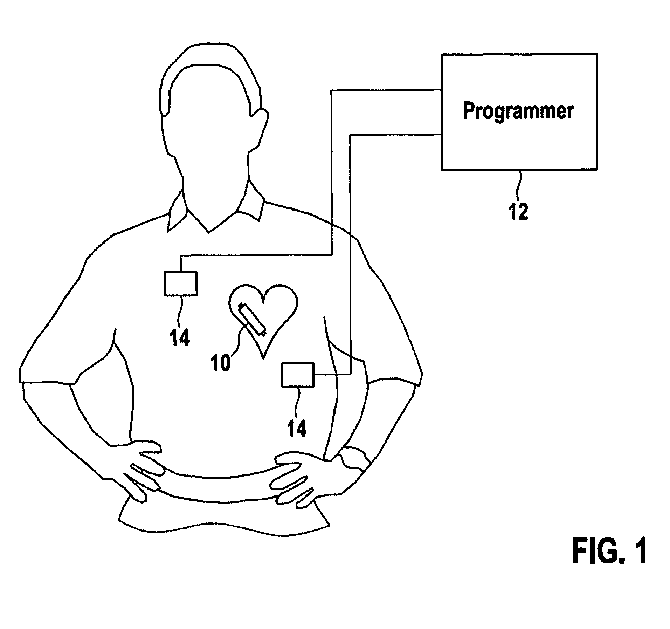

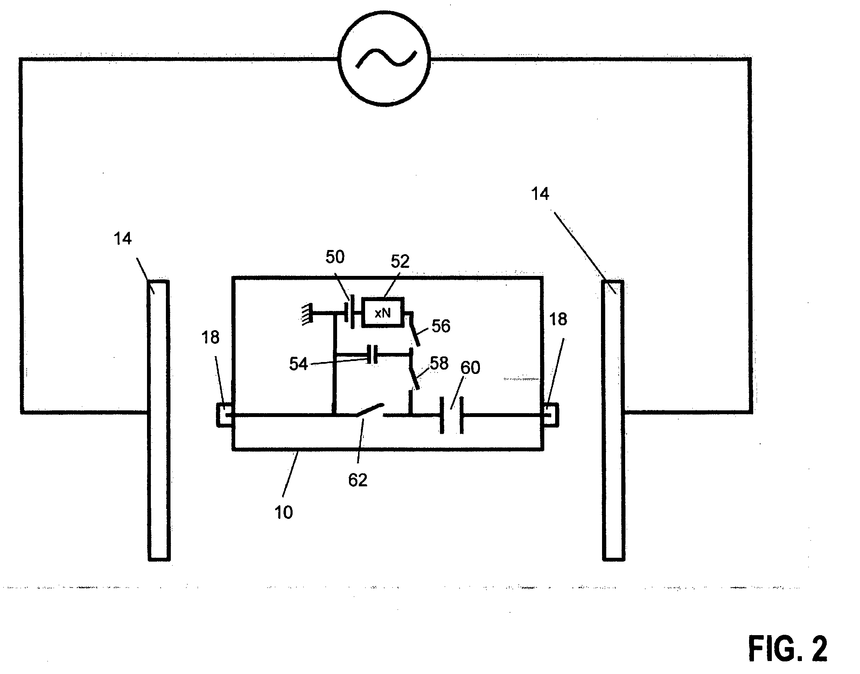

In one embodiment, utilizing an implantable pacemaker as an implantable stimulation device 10, a programmer/communication device as external device 12 uses cutaneous electrodes 14 placed on either side of the heart as shown in Fig. 1. In one embodiment, the external device 12 detects changes of a local electric field in the body. In particular the external device 12 detects changes of a local electric field in the body caused by a stimulation pulse and/or autoshort pulse. The external device 12 can induce an oscillating electric field through the electrodes 14 between 50 kHz to 1 MHz, preferably between 300 to 500 kHz, at a known voltage or current. The stimulation device 10 implanted in the heart is located between the at least two cutaneous electrodes 14; as shown in Fig. 2.

The implantable stimulation device 10 comprises a battery 50 with limited capacity. Optionally, to multiply the voltage V_{bat} of battery 50 to generate a voltage needed for a stimulation pulse, a voltage multiplier 52 is provided. Further, a stimulation pulse capacitor 54 is provided that can be charged when connected to voltage multiplier 52 or battery 50. To enable charging of the stimulation pulse capacitor 54 a charging control switch 56 is provided. The stimulation pulse capacitor 54 is charged when charging control switch 56 is closed.

To deliver the charge from stimulation pulse capacitor 54 to electrodes 18 and to thus deliver a stimulation pulse, a stimulation-pulse-switch 58 is provided. When the charging control switch 56 is opened and the stimulation-pulse-switch 58 is closed, current can flow to electrodes 18 via a blocking capacitor C_{block} 60. Via stimulation-pulse-switch 58 the duration of a stimulation pulse period can be controlled. The stimulation pulse period lasts as long as stimulation-pulse-switch 58 is closed.

After delivery of a stimulation pulse when the stimulation-pulse-switch 58 is opened, charge remaining on blocking capacitor 60 can be discharged via autoshort switch 62 and electrodes 18. Thus an autoshort pulse is generated that dissipates charge on the blocking capacitor remaining from the stimulation pulse. The autoshort pulse is thus controlled by autoshort switch 62.

According to one embodiment, autoshort switch 62 is controlled by data communication means not shown in Figure 2.

Utilizing the inherent charge present on the blocking capacitor 60 after delivery of a stimulation pulse would effect an observable change of an electric field that can be used for data transmission. Normally after delivery of a stimulation pulse, the residual charge on the blocking capacitor 60 is removed by shorting the stimulation electrodes 18 together (Autoshort). The voltage left on the capacitor 60 after delivery of a stimulation pulse is not stimulating voltage, rather, it is unused. It is a by-product of pacing and detrimental to leads and tissue. It must be removed to prevent for example electrode corrosion and therefore autoshort switch 62 is provided. By modulating the shorting process to encode data, a detectable data transmission signal can be produced. The modulation can comprise any suitable modulation and coding, for example amplitude modulation (AM).

The modulated residual charge creates a large change in the local field around the implantable stimulation device 10 that causes large voltage changes as sensed by the external device 12. The external device 12 can impart an electric field on the body to create a carrier for the implanted device to use for transmission. Modulating the charge present after delivery of a stimulation pulse causes the local field to change within the frequency range of a receiving lock-in amplifier of the external device 12. A synchronization of the modulation to the frequency of the externally imparted electric field improves signal to noise ratio. The change in the field produced by the external device 12, which includes a receiver, causes communication. This communication is termed pseudo-passive because charge is used to change the local field, whereas passive only uses a switch.

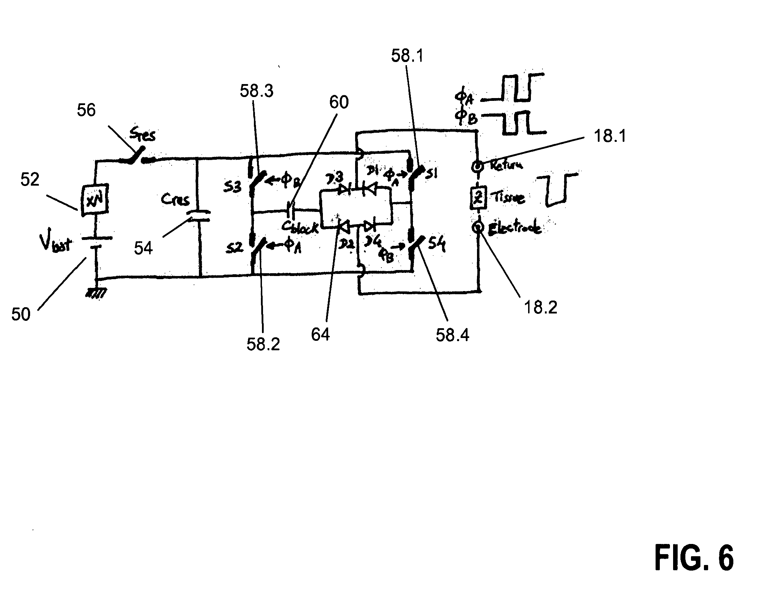

This method can be extended for use with stimulation pulses. Fast modulation of stimulation-pulse peak amplitudes, without return to baseline, can be achieved using a circuit that includes a blocking capacitor 60, a rectifier diode bridge 64, and four switches 58.1, 58.2, 58.3 and 58.4 as shown in Figure 6. The switches 58.1, 58.2, 58.3 and 58.4 allow bi-directional current flow through the blocking capacitor 60, and such current is unidirectionally delivered to tissue via the rectifier diode bridge 64. The blocking capacitor 60 will charge and discharge within the delivery of a stimulation pulse and can be sized to provide the desired modulation of the peak amplitude of the stimulation pulse. This approach generally results in a smaller blocking capacitor 60 which is an advantage for volume-constrained applications such an iLP.

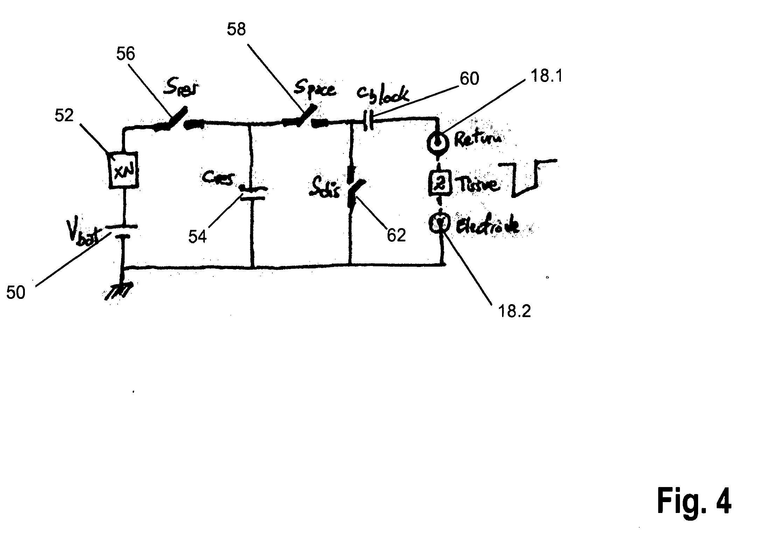

Passive charge balancing of a pacing pulse involves the connection of analog switch(es) that allow discharging the blocking capacitor(s) 60 through tissue, returning charge for neutrality purpose. Figure 4 shows a schematic representation of a pacing circuit. In such circuit, capacitor Cᵣₑₛ 54 is charged via switch Sᵣₑₛ 56 to a multiple N of the battery voltage V_{bat} 50 (N can be 1). AC-coupled stimulation, via capacitor C_{block} 60, occurs by opening switch Sᵣₑₛ 56 and closing switch S_{pace} 58, connecting capacitor Cᵣₑₛ 54 in series with C_{block} 60 and tissue (represented by Z) between electrodes 18. Capacitor C_{block} 60 will then charge during the delivery of the stimulation pulse and such charge must flow in the opposite direction following the stimulation pulse (opening of switch S_{pace} 58) to allow for charge-balanced stimulation. This can be achieved by closing switch S_{dis} 62 for a finite time that will depend on the Re(Z)xC_{block} time constant.

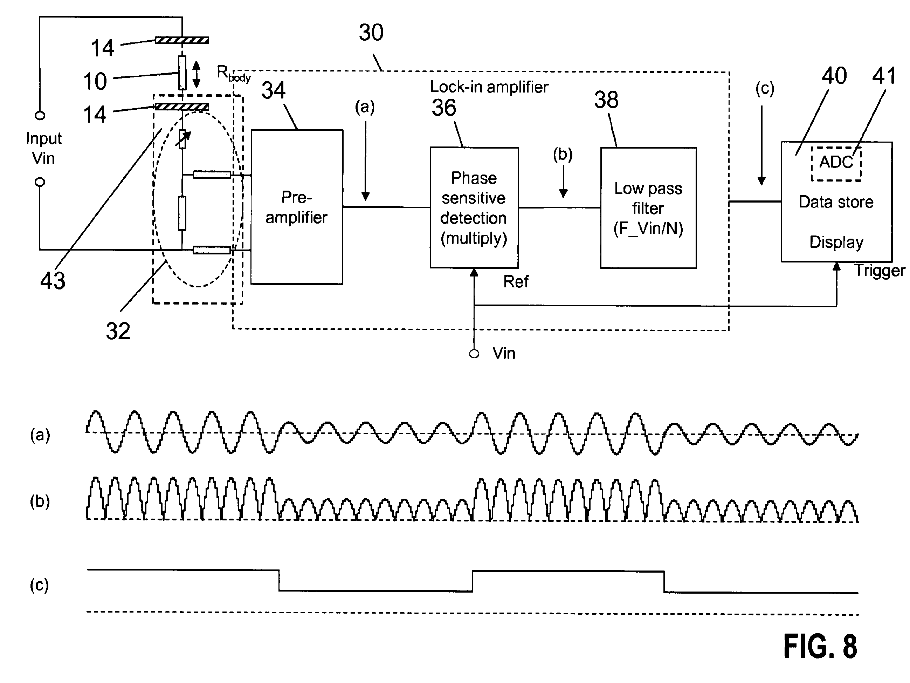

Fast connection/disconnection of switch S_{dis} 62 will cause interruption of the balancing autoshort pulse which in turn will create voltage glitches between the electrodes 18. Such glitches can be detected using a lock-in amplifier as disclosed in Figure 8.

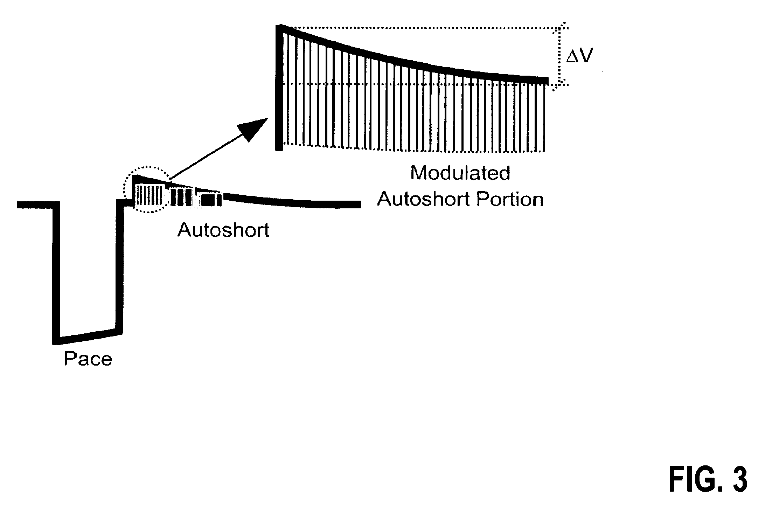

As shown in Figure 3, transmission during the balancing phase can be divided into different sections. Each section is selected according to a maximum ΔV discharge of the blocking capacitor(s) that allows decoding the glitches within a section using the same integration time for the receiving lock-in amplifier. Consecutive transmission sections may have increasing integration times until charge balancing complete. Transition from one section to the next may be achieved by simple start and stop bits in the link layer.

The implantable stimulation device 10 can measure the lead impedance and compare such value against those in a non-volatile table stored in the device's memory to support determination of each section's frequency carrier. This information can be transmitted to the external receiver during synchronization, or extracted by such, to determine the corresponding integration times to be used.

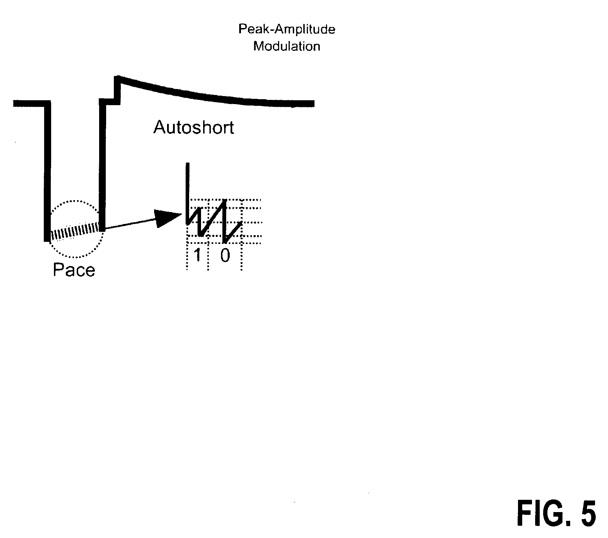

Figure 5 shows modulation of the stimulation-pulse peak amplitude instead, for data transmission during delivery of a stimulation pulse. Such modulation can be achieved with the circuit of Figure 6.

As in the embodiment of Figure 4, capacitor Cᵣₑₛ 54 is charged to a multiple N of the battery voltage V_{bat}. To cause delivery of a stimulation pulse, switch Sᵣₑₛ 56 is opened and switches 58.1, 58.2, 58.3 and 58.4 are opened and closed in an H-bridge type of configuration. For example, during the high-phase of Φ_{A}, switches 58.1 and 58.2 are closed simultaneously which makes diodes D1 and D2 to conduct flowing current from the first electrode 18.1 to the second electrode 18.2. C_{block} will then charge in the direction of diode D2 and switch 58.2, reducing (approximately linearly) the voltage across Z. The former is dimensioned to provide the desired amplitude/timing modulation shown in Figure 5 (for a range ofRe(Z)).

In the opposite phase, i.e. high ΦB and low ΦA, switches 58.1 and 58.2 are opened and switches 58.3 and 58.4 are closed. The accumulated voltage on C_{block} 60 will instantaneously add to the stimulation pulse peak voltage, making the transitions shown in the zoom of Figure 5. Current will flow through switch 58.3 → capacitor C_{block} 60 → diode D3 → diode D4 → switch 58.4 thus discharging C_{block} 60.

In a preferred embodiment, diodes D1...D4 in Figure 6 are replaced by switching elements for high efficiency implementation.

Combination of the schematized circuits of Figure 4 and 6 can be realized to achieve data transmission during both the stimulation pulse period and the autoshort period.

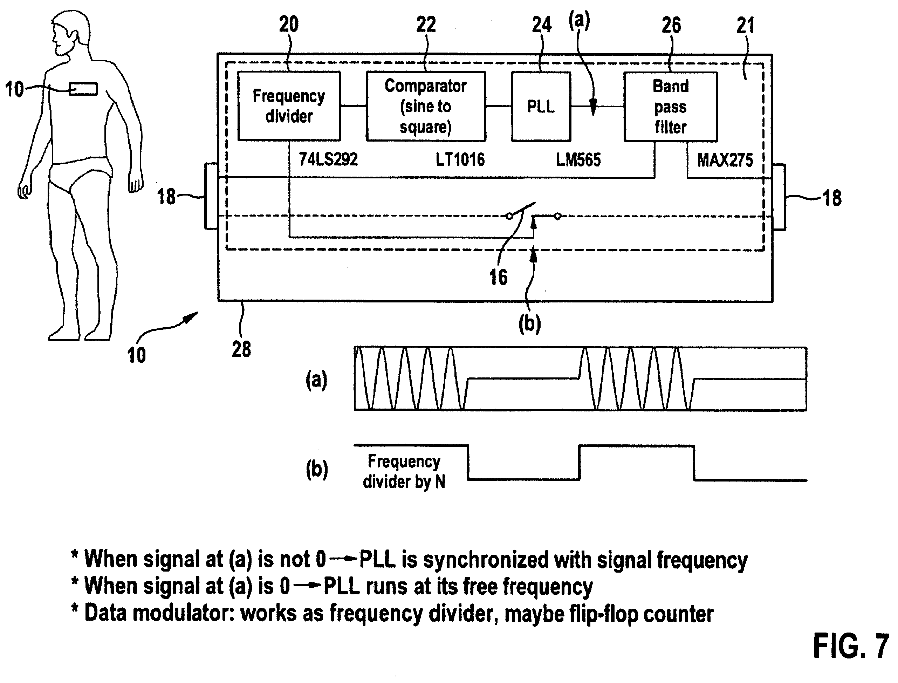

Referring to Fig. 7, the implantable stimulation device 10 comprises two (implantable) electrodes 18 that contact body tissue surrounding the implantable stimulation device 10 in its implanted state. Switch 62 and switches 58.1, 58.2, 58.3 and 58.4, respectively, are represented by single switch 16 in Fig. 7.

The at least two electrodes 18 of the implantable stimulation device 10 preferably are arranged on the external surface of a hermetically sealed housing 28 encapsulating the implantable stimulation device 10. According to a preferred embodiment, parts of the housing 28 itself form the at least two electrodes 18. The electrodes 18 can also be formed by a tip electrode 18 located at a tip of the implantable stimulation device 10 and a ring electrode 18' located on the circumference of the implantable stimulation device 10 (not shown).

In order to control switching of the switch or switches represented by switch 16, the implantable stimulation device 10 further comprises a frequency divider 20 connected to a sine-to-square converting comparator 22 that in turn is connected to a phase-locked loop (PLL) 24. Phase-locked loop 24 is connected to the electrodes 18 via band-pass filter 26. Phase-locked loop 24 and frequency divider 20 are part of a switch control of implantable stimulation device 10.

The field induced between the cutaneous electrodes 14 is sensed by the implantable stimulation device 10. The implantable stimulation device 10 locks in the frequency of the electric field using the phase-locked loop 24. Once the implantable stimulation device 10 is locked on to the frequency of the external device's induced field, it can activate the switch 16 between the two electrodes 18 that are in the field (see Figs. 2 and 4) in synch with the frequency of the electric field.

In more detail: The implantable stimulation device 10 receives the imposed oscillatory electric signal as input signal that is detected via the electrodes 18 . Thus, the implantable stimulation device 10 has an input sine signal that can be detected as an alternating voltage across electrodes 18 or across a resistor. This input sine signal is band-pass filtered by band-pass filter 26. A representation of that band-pass filtered signal is shown in Fig. 7 (a).

The band-pass filtered input sine signal is fed to the phase-locked loop (PLL) 24 that locks in the frequency of the input sine signal. Phase-locked loop 24 puts out a synchronized sine signal to a comparator 22 that converts the sine signal Fig. 7 (a) to a square signal Fig. 7(b). The square signal thus generated is fed to frequency divider 20 that generates a clock signal for switching the switching means 16. The clock signal thus generated has a frequency corresponding to a fraction of the frequency of the oscillatory electric field wherein the fraction is determined by a frequency division factor applied by frequency divider 20. The clock signal frequency is preferably in a range of 0.1 to 50 kHz, such as a range of 1 to 20 kHz, more preferably in a range of 7 to 9 kHz, most preferably the clock signal frequency is 8 kHz.

The actual switching of the switch or switches represented by switch 80 further depends on data that are to be transmitted from the implantable stimulation device 10 to the external device 12. The data to be transmitted is coded and the code determines the actual sequence of switching of the switch or switches represented by switch 80.

Frequency divider 20 can be realized as a flip-flop counter.

The change of an electrical field caused by switching the switch 62 and/or switches 58.1, 58.2, 58.3 and 58.4 represented by switch 80 can be sensed by external device 12.

One embodiment of data transmission from the implantable stimulation device 10 to the external device 12 thus can be summarized as follows:
Generate a local electric field in the body by discharging a capacitor to the body tissue → switch on/off the switch or switches represented by switch 16 in implantable device 10 to cause changes of the body electric field → detect change by external device 12.

Another embodiment of data transmission from the implantable stimulation device 10 to the external device 12 thus can be summarized as follows:
Apply signal (oscillatory electric field) → propagate in body → switch on/off the switch or switches represented by switch 16 in implantable device 10 to cause changes of the body electric field → detect change by external device 12.

The switch control of the implantable stimulation device 10 thus receives an input sine signal by detecting a voltage across electrodes 18. The switch control of the implantable stimulation device 10 according to the embodiment of Fig. 7 comprises band-pass filter 26, phase-locked loop 24 to lock in the frequency of the input sine signal, comparator 22 to convert the sine signal to a square signal, a flip-flop counter that acts as frequency divider 20 that control switching means 16. Switching means 16 has a small on-resistance.

The changes of the electric field caused by the implantable stimulation device 10 shown in Fig. 4 and 6 can be detected by the external device 12. As shown in Fig. 8, the external device 12 essentially comprises a lock-in amplifier 30 that generates an output signal (signal of Fig. 8 (c)) that represents the signal transmitted by implantable stimulation device 10 by way of electric field changes. Lock-in amplifier 30 uses the signal imposed on a body by means of cutaneous electrodes 14 as a reference signal. For this purpose, a network of resistors 32 is provided that causes a voltage drop representing the signal (the oscillatory electric field) imposed on a body via cutaneous electrodes 14.

This signal is amplified by pre-amplifier 34 of lock-in amplifier 30.

The amplified signal sensed via sensing means 43, i.e. cutaneous electrodes 14 and the resistor network 32 is fed to an AM demodulator comprising phase-sensitive detector 36 and further fed to a low-pass filter 38, as depicted in Fig. 8. The amplified input signal sensed via cutaneous electrodes 14 and the resistor network 32 is represented as signal (a) in Fig. 8 (a). The output signal of the phase-sensitive detector 36 is depicted as signal (b) in Fig. 8 (b). The low-pass filtered output signal of lock-in amplifier 30 is depicted in Fig. 8(c) as signal (c). Signal (c) corresponds to the signal generated by implantable stimulation device 10 and thus represents data to be transmitted from implantable stimulation device 10 to external device 12. This signal can be analog-to-digital converted and stored. Block 40 in Fig. 8 represents an analog-to-digital converter (ADC) 41, a memory for data storage and a display of external device 12.

For the detection of electric field changes of the body caused by the implantable stimulation device 10, the external device 12 thus comprises a lock-in amplifier 30 that is configured to use the input as reference signal, comprising an AM demodulator which in turn comprises a precision rectifier and a low-pass filter 38. The low-pass filtered signal is fed to an analog-to-digital converter 41.

The communication from implantable stimulation device 10 to external device 12 thus can be understood as follows.

As the implantable stimulation device 10 alternatively shorts and opens the switch 62 and/or switches 58.1, 58.2, 58.3 and 58.4 represented by switch 16, the electric field between the external device electrodes 14 is slightly changed or modulated. The external device 12 can sense the change of the electric field by measuring how much voltage or current is imparted on the electrodes 14 to create the oscillator electric field between the external device electrodes 14.

The external device 12 does this using a lock-in amplifier 30 that is synchronized to the electric field frequency and phase. As the implantable stimulation device 10 modulates the electric field between the external device electrodes 14, the external device 12 integrates the changing current or voltage. The integration allows a very small change in sourced voltage or current to be detected using amplitude modulation.

Communication can occur at approximately 1/10 to 1/100th of the modulation frequency of the imposed oscillatory electric field. This allows for ∼10-100 cycles of the electric field to be integrated to determine the imparted current or voltage.

A communication from external device 12 to implantable stimulation device 10 can be done as follows:

The imposed oscillatory electric field has a fundamental frequency that the implantable stimulation device 10 uses to lock onto. The fundamental frequency is also used as a carrier frequency to send modulated data to the implantable stimulation device 10. The external device 12 modulates data, using frequency modulation or amplitude modulation, on top of the carrier imparted electric field. The implantable stimulation device 10 decodes the modulated data sensed through the electrodes 18.

The present invention does not require the implantable stimulation device 10 to actively transmit data using its own power in the case of impedance modulation embodiment. It simply modulates a field imparted on it by an external device 12. In both embodiments, the resulting drain on the implantable stimulation device's battery is negligible. Because of the small power consumption, it is possible to transmit more data to the external device 12.

Modulating a local electric field by means as described herein above allows for improved data communication than data communication provided by causing pure impedance changes. The changes in the local electric field produce much larger changes in the sensed voltage at the receiver than pure impedance changes between electrodes of the implantable device. Because of the large sensed difference, a shortened integration time is possible. By shortening the integration time of the lock-in amplifier, higher data rates are achieved.

Further, utilizing this method may enable post-stimulation-pulse transmission of data.

## Claims

1. Implantable stimulation device (10) comprising stimulation means and data communication means, said stimulation means comprising or is connectable to at least two electrodes (18) that are configured to allow delivery of stimulation pulses, said stimulation means further comprising a DC-blocking capacitor (60) that is connected in series with a high voltage source (50, 52) and one of the electrodes (18); **characterized in that**
said stimulation means comprises the high-voltage source (50, 52) that is connected to the electrodes via at least one stimulation-pulse-switch (58; 58.1, 58.2, 58.3, 58.4) that controls delivery of a pacing pulse,
said stimulation means further comprises an autoshort switch (62) that is arranged and configured to allow discharging the DC-blocking capacitor (60) via the electrodes (18) when the autoshort switch (62) is closed; and
said data communication means comprises data transmission control means that are connected to the autoshort switch (62) and/or the stimulation-pulse-switch (58; 58.1, 58.2, 58.3, 58.4) and that are configured to alternatingly open and close the autoshort switch (62) or the stimulation-pulse-switch (58; 58.1, 58.2, 58.3, 58.4), respectively, during an autoshort period following the delivery of a stimulation pulse or during a stimulation pulse period, respectively, to modulate an autoshort pulse or a stimulation pulse peak amplitude for the purpose of data communication, respectively.

2. Implantable stimulation device (10) according to claim 1, wherein the data transmission control means are configured to control switching of the autoshort switch (60) and/or the stimulation-pulse-switch (58; 58.1, 58.2, 58.3, 58.4) so as to generate an oscillatory electric field that is phase-synchronized with an oscillatory electric field imposed on body tissue surrounding the implantable device (10) when the implantable device (10) is operating in its implanted state.

3. Implantable stimulation device (10) according to claim 1 or 2, comprising four switches (58.1, 58.2, 58.3, 58.4) and a rectifier diode bridge (64) that are arranged and configured to allow bi-directional current flow through the blocking capacitor (60) to generate a stimulation pulse having a modulated peak amplitude that is unidirectionally delivered to tissue via the rectifier diode bridge (64).

4. Implantable stimulation device (10) according to any one or more of claims 1 to 3, wherein the data transmission control means are connected to a switch control that is configured to sense an oscillatory electric field imposed on body tissue surrounding the implantable device (10) when the implantable device (10) is in its implanted state.

5. Implantable device (10) according to claim 4, wherein the switch control comprises a phase-locked loop (PLL) (24) and a frequency divider (20), wherein the phase-locked loop (24) is configured to lock in a frequency of an oscillatory electric field imposed on body tissue surrounding the implantable device (10) when the implantable device (10) is in its implanted state, and wherein the frequency divider (20) is connectable to the phase-locked loop (24) and is configured to divide a frequency signal put out by the phase-locked loop (24).

6. Implantable device (10) according to claim 5, wherein the switch control is connectable to the at least two electrodes (18, 18') and is configured to sense an oscillatory electric field imposed on body tissue via the at least two electrodes (18, 18').

7. Implantable device (10) according to claim 6, wherein the switch control comprises a band-pass filter (26), wherein the band-pass filter (26) filters a signal fed to the phase-locked loop (24).

8. Data communication system including an implantable stimulation device (10) according to at least one of the claims 1 to 7 and further comprising an external device (12) that comprises or can be connected to at least two cutaneous electrodes (14), wherein the external device (12) further comprises external field generating means that are configured to generate an oscillatory electric field to be transcutaneously imposed on a body via the at least two cutaneous electrodes (14), wherein the external device (12) further comprises means (43) for sensing alterations of body impedance and/or a local electric field generated by the implantable device (10) when the implantable device (10) is in its implanted state.

9. Data communication system according to claim 8, wherein the external device (12) comprises a lock-in amplifier (30), an AM demodulator for demodulating amplitude-modulated signals and an analog-to-digital converter (41), wherein the analog-to-digital converter (41) is operatively connected to the AM demodulator and the lock-in amplifier (30), and wherein the analog-to-digital converter (41) puts out a signal that represents a signal transmitted by the implantable device (10).

10. Method of communicating data from an implantable stimulation device (10) to an external device (12), said method comprising:
- imposing an oscillatory electric field in body tissue encompassing an implantable stimulation device (10),
- sensing the imposed oscillatory electric field by means of the implantable stimulation device (10),
- altering the oscillatory electric field by means of the implantable stimulation device (10)
- by modulating an autoshort pulse
- sensing the change of the local electric field caused by the modulation of the autoshort pulse and/or a stimulation pulse peak amplitude, respectively, by means of an external device (12) having or being connected to at least two cutaneous electrodes (14).

11. Method according to claim 10, wherein the step of altering the oscillatory electric field by means of the implantable stimulation device (10) is performed by means of alternatingly open and close an autoshort switch (62) or the stimulation-pulse-switch (58; 58.1, 58.2, 58.3, 58.4), respectively, during an autoshort period following the delivery of a stimulation pulse to modulate an autoshort pulse or a stimulation pulse peak amplitude, respectively.

12. Method according to at least one of the claims 10 or 11, wherein the step of sensing the change of a local electric field comprises sensing a current or voltage over cutaneous electrodes (14) and integrating a sensed voltage or current over a number of cycles of the oscillatory electric field, said number of cycles corresponding to a frequency division factor of a frequency divider (20) of the implantable device (10).

## Patentansprüche

1. Implantierbare Stimulationsvorrichtung (10), die eine Stimulationseinrichtung und eine Datenkommunikationseinrichtung aufweist, wobei die genannte Stimulationseinrichtung (mit) wenigstens zwei Elektroden (18) aufweist oder verbindbar ist, die konfiguriert sind, um die Abgabe von Stimulationsimpulsen zu ermöglichen, wobei die genannte Stimulationseinrichtung ferner einen Gleichstrom blockierenden Kondensator (60) aufweist, der mit einer Quelle hoher Spannung (50, 52) und einer der Elektroden (18) in Reihe geschaltet ist;
**dadurch gekennzeichnet, dass**:
die genannte Stimulationseinrichtung die Quelle hoher Spannung (50, 52) aufweist, die über wenigstens einen Stimulationsimpulsschalter (58; 58.1, 58.2, 58.3, 58.4) mit den Elektroden verbunden ist, der die Abgabe eines Schrittmacherimpulses steuert,
die genannte Stimulationseinrichtung ferner einen Autoshort-Schalter (62) aufweist, der angeordnet und konfiguriert ist, um das Entladen des Gleichstrom blockierenden Kondensators (60) über die Elektroden (18) zu ermöglichen, wenn der Autoshort-Schalter (62) geschlossen wird; und
die genannte Datenkommunikationseinrichtung Datenübertragungssteuereinrichtungen aufweist, die mit dem Autoshort-Schalter (62) und/oder dem Stimulationsimpulsschalter (58; 58.1, 58.2, 58.3, 58.4) verbunden sind und die konfiguriert sind, um den Autoshort-Schalter (62) bzw. den Stimulationsimpulsschalter (58; 58.1, 58.2, 58.3, 58.4) während einer Autoshort-Periode nach der Abgabe eines Stimulationsimpulses bzw. während einer Stimulationsimpulsperiode abwechselnd zu öffnen und zu schließen, um einen Autoshort-Impuls bzw. eine Stimulationsimpuls-Spitzenamplitude zum Zweck der Datenkommunikation zu modulieren.

2. Implantierbare Stimulationsvorrichtung (10) nach Anspruch 1, wobei die Datenübertragungssteuereinrichtungen konfiguriert sind, um das Schalten des Autoshort-Schalters (60) und/oder des Stimulationsimpulsschalters (58; 58.1, 58.2, 58.3, 58.4) zu steuern, um ein oszillierendes elektrisches Feld zu erzeugen, das mit einem oszillierenden elektrischen Feld phasensynchronisiert ist, mit dem die implantierbare Vorrichtung (10) umgebendes Gewebe beaufschlagt wird, wenn die implantierbare Vorrichtung (10) in ihrem implantierten Zustand betrieben wird.

3. Implantierbare Stimulationsvorrichtung (10) nach Anspruch 1 oder 2, die vier Schalter (58.1, 58.2, 58.3, 58.4) und eine Gleichrichter-Diodenbrücke (64) aufweist, die angeordnet und konfiguriert sind, um einen bidirektionalen Stromfluss durch den blockierenden Kondensator (60) zum Erzeugen eines Stimulationsimpulses mit einer modulierten Spitzenamplitude zu erzeugen, der über die Gleichrichter-Diodenbrücke (64) unidirektional an Gewebe abgegeben wird.

4. Implantierbare Stimulationsvorrichtung (10) nach einem oder mehr der Ansprüche 1 bis 3, wobei die Datenübertragungssteuereinrichtungen mit einer Schaltersteuerung verbunden sind, die zum Erfassen eines oszillierenden elektrischen Felds konfiguriert ist, mit dem die implantierbare Vorrichtung (10) umgebendes Gewebe beaufschlagt wird, wenn die implantierbare Vorrichtung (10) in ihrem implantierten Zustand ist.

5. Implantierbare Vorrichtung (10) nach Anspruch 4, wobei die Schaltersteuerung eine Phasenregelschleife (PLL) (24) und einen Frequenzteiler (20) aufweist, wobei die Phasenregelschleife (24) konfiguriert ist, um eine Frequenz eines oszillierenden elektrischen Felds einzurasten, mit dem die implantierbare Vorrichtung (10) umgebendes Gewebe beaufschlagt wird, wenn die implantierbare Vorrichtung (10) in ihrem implantierten Zustand ist, und wobei der Frequenzteiler (20) mit der Phasenregelschleife (24) verbindbar ist und zum Teilen eines von der Phasenregelschleife (24) ausgegebenen Frequenzsignals konfiguriert ist.

6. Implantierbare Vorrichtung (10) nach Anspruch 5, wobei die Schaltersteuerung mit den wenigstens zwei Elektroden (18, 18') verbindbar ist und zum Erfassen eines oszillierenden elektrischen Felds konfiguriert ist, mit dem Körpergewebe über die wenigstens zwei Elektroden (18, 18') beaufschlagt wird.

7. Implantierbare Vorrichtung (10) nach Anspruch 6, wobei die Schaltersteuerung ein Bandpassfilter (26) aufweist, wobei das Bandpassfilter (26) ein in die Phasenregelschleife (24) eingespeistes Signal filtert.

8. Datenkommunikationssystem, das eine implantierbare Stimulationsvorrichtung (10) nach wenigstens einem der Ansprüche 1 bis 7 beinhaltet und das ferner eine externe Vorrichtung (12) aufweist, die (mit) wenigstens zwei Hautelektroden (14) aufweist oder verbunden sein kann, wobei die externe Vorrichtung (12) ferner externe Felderzeugungseinrichtungen aufweist, die konfiguriert sind, um ein oszillierendes elektrisches Feld zu erzeugen, mit dem ein Körper über die wenigstens zwei Hautelektroden (14) transkutan beaufschlagt wird, wobei die externe Vorrichtung (12) ferner eine Einrichtung (43) zum Erfassen von Veränderungen der Körperimpedanz und/oder eines lokalen elektrischen Felds aufweist, das von der implantierbaren Vorrichtung (10) erzeugt wird, wenn die implantierbare Vorrichtung (10) in ihrem implantierten Zustand ist.

9. Datenkommunikationssystem nach Anspruch 8, wobei die externe Vorrichtung (12) einen Trägerfrequenzverstärker (30), einen AM-Demodulator zum Demodulieren von amplitudenmodulierten Signalen und einen Analog-Digital-Umsetzer (41) aufweist, wobei der Analog-Digital-Umsetzer (41) funktionell mit dem AM-Demodulator und dem Trägerfrequenzverstärker (30) verbunden ist und wobei der Analog-Digital-Umsetzer (41) ein Signal ausgibt, das ein von der implantierbaren Vorrichtung (10) übertragenes Signal repräsentiert.

10. Verfahren zur Datenkommunikation von einer implantierbaren Stimulationsvorrichtung (10) zu einer externen Vorrichtung (10), wobei das genannte Verfahren Folgendes aufweist:
- Beaufschlagen von eine implantierbare Stimulationsvorrichtung (10) umschließendem Körpergewebe mit einem oszillierenden elektrischen Feld,
- Erfassen des durch Beaufschlagen angelegten oszillierenden elektrischen Felds mithilfe der implantierbaren Stimulationsvorrichtung (10),
- Ändern des oszillierenden elektrischen Felds mithilfe der implantierbaren Stimulationsvorrichtung (10) durch Modulieren eines Autoshort-Impulses,
- Erfassen der Änderung des lokalen elektrischen Felds, die durch die Modulation des Autoshort-Impulses und/bzw. einer Stimulationsimpuls-Spitzenamplitude mithilfe einer externen Vorrichtung (12) verursacht wird, die wenigstens zwei Hautelektroden (14) hat oder verbunden ist.

11. Verfahren nach Anspruch 10, wobei der Schritt des Änderns des oszillierenden elektrischen Felds mithilfe der implantierbaren Stimulationsvorrichtung (10) mithilfe von abwechselndem Öffnen und Schließen eines Autoshort-Schalters (62) bzw. des Stimulationsimpulsschalters (58; 58.1, 58.2, 58.3, 58.4) während einer Autoshort-Periode nach der Abgabe eines Stimulationsimpulses zum Modulieren eines Autoshort-Impulses bzw. einer Stimulationsimpuls-Spitzenamplitude durchgeführt wird.

12. Verfahren nach wenigstens einem der Ansprüche 10 oder 11, wobei der Schritt des Erfassens der Änderung eines lokalen elektrischen Felds das Erfassen eines Stroms oder einer Spannung über Hautelektroden (14) und das Integrieren einer/eines erfassten Spannung oder Stroms über eine Anzahl von Zyklen des oszillierenden elektrischen Felds aufweist, wobei die genannte Anzahl von Zyklen einem Frequenzteilungsfaktor eines Frequenzteilers (20) der implantierbaren Vorrichtung (10) entspricht.

## Revendications

1. Dispositif de stimulation implantable (10) comprenant des moyens de stimulation et des moyens de communication des données, lesdits moyens de stimulation comprenant ou pouvant être reliés à au moins deux électrodes (18) qui sont configurées pour permettre une délivrance d'impulsions de stimulation, lesdits moyens de stimulation comprenant en outre un condensateur de blocage du courant continu (60) qui est relié en série avec une source de tension élevée (50, 52) et à l'une des électrodes (18) ;
**caractérisé en ce que**
lesdits moyens de stimulation comprennent la source de tension élevée (50, 52) qui est reliée aux électrodes via au moins un commutateur d'impulsion de stimulation (58 ; 58.1, 58.2, 58.3, 58.4) qui contrôle la délivrance d'une impulsion de stimulation,
lesdits moyens de stimulation comprennent en outre un commutateur d'autoshort (62) qui est disposé et configuré pour permettre le déchargement du condensateur de blocage du courant continu (60) via les électrodes (18) lorsque le commutateur d'autoshort (62) est fermé ; et
lesdits moyens de communication des données comprennent des moyens de contrôle de la transmission des données qui sont connectés au commutateur d'autoshort (62) et/ou au commutateur d'impulsion de stimulation (58 ; 58.1, 58.2, 58.3, 58.4) et qui sont configurés pour ouvrir et fermer en alternance le commutateur d'autoshort (62) ou le commutateur d'impulsion de stimulation (58 ; 58.1, 58.2 ; 58.3, 58.4), respectivement, au cours d'une période d'autoshort suivant la délivrance d'une impulsion de stimulation ou au cours d'une période d'impulsion de stimulation, respectivement, pour moduler une impulsion d'autoshort ou une amplitude du pic d'une impulsion de stimulation dans le but de la communication de données, respectivement.

2. Dispositif de stimulation implantable (10) selon la revendication 1, dans lequel les moyens de contrôle de la transmission des données sont configurés pour contrôler la commutation du commutateur d'autoshort (60) et/ou le commutateur d'impulsion de stimulation (58 ; 58.1, 58.2, 58.3, 58.4) afin de générer un champ électrique oscillant qui est synchronisé en phase avec un champ électrique oscillant imposé au tissu du corps entourant le dispositif implantable (10) lorsque le dispositif implantable (10) est fonctionnel dans son état implanté.

3. Dispositif de stimulation implantable (10) selon les revendications 1 ou 2, comprenant quatre commutateurs (58.1, 58.2, 58.3, 58.4) et un pont redresseur à diode (64) qui sont disposés et configurés pour permettre une circulation du courant bidirectionnelle à travers le condensateur de blocage (60) pour générer une impulsion de stimulation ayant une amplitude de pic modulée qui est délivrée de manière unidirectionnelle au tissu via le pont redresseur à diode (64).

4. Dispositif de stimulation implantable (10) selon l'une quelconque ou plusieurs des revendications 1 à 3, dans lequel les moyens de contrôle de la transmission des données sont reliés à un contrôle de la commutation qui est configuré pour détecter un champ électrique oscillant imposé au tissu du corps entourant le dispositif implantable (10) lorsque le dispositif implantable (10) est dans son état implanté.

5. Dispositif implantable (10) selon la revendication 4, dans lequel le contrôle de la commutation comprend une boucle à asservissement de phase (PLL) (24) et un diviseur de fréquence (20), où la boucle à asservissement de phase (24) est configurée pour asservir une fréquence d'un champ électrique oscillant imposé au tissu du corps entourant le dispositif implantable (10) lorsque le dispositif implantable (10) est dans son état implanté, et dans lequel le diviseur de fréquence (20) peut être raccordé à la boucle à asservissement de phase (24) et est configuré pour diviser un signal de fréquence sortant de la boucle à asservissement de phase (24).

6. Dispositif implantable (10) selon la revendication 5, dans lequel le contrôle de la commutation peut être raccordé aux au moins deux électrodes (18, 18') et est configuré pour détecter un champ électrique oscillant imposé au tissu du corps via au moins les deux électrodes (18, 18').

7. Dispositif implantable (10) selon la revendication 6, dans lequel le contrôle de la commutation comprend un filtre passe bande (26), dans lequel le filtre passe bande (26) filtre un signal envoyé à la boucle à asservissement de phase (24).

8. Système de communication de données comprenant un dispositif de stimulation implantable (10) selon au moins une des revendications 1 à 7 et comprenant en outre un dispositif externe (12) qui comprend, ou qui peut être relié, à au moins deux électrodes cutanées (14), où le dispositif externe (12) comprend en outre des moyens de génération d'un champ externe qui sont configurés pour générer un champ électrique oscillant devant être imposé par voie transcutanée à un corps via les au moins deux électrodes cutanées (14), où le dispositif externe (12) comprend en outre des moyens (43) pour capter des altérations de l'impédance du corps et/ou d'un champ électrique local généré par le dispositif implantable (10) lorsque le dispositif implantable (10) est dans son état implanté.

9. Système de communication de données selon la revendication 8, dans lequel le dispositif externe (12) comprend un amplificateur synchrone (30), un démodulateur AM pour démoduler des signaux modulés en amplitude et un convertisseur analogique-numérique (41), où le convertisseur analogique-numérique (41) est relié de manière fonctionnelle au démodulateur AM et à l'amplificateur synchrone (30), et où le convertisseur analogique-numérique (41) délivre un signal qui représente un signal transmis par le dispositif implantable (10).

10. Procédé pour communiquer des données à partir d'un dispositif de stimulation implantable (10) en direction d'un dispositif externe (12), ledit procédé comprenant :
- l'imposition d'un champ électrique oscillant dans un tissu du corps entourant un dispositif de stimulation implantable (10),
- la détection du champ électrique oscillant imposé au moyen du dispositif de stimulation implantable (10),
- l'altération du champ électrique oscillant au moyen du dispositif de stimulation implantable (10)
- par la modulation d'une impulsion d'autoshort,
- la détection du changement du champ électrique local dû à la modulation de l'impulsion d'autoshort et/ou de l'amplitude du pic d'une impulsion de stimulation, respectivement, au moyen d'un dispositif externe (12) ayant, ou étant relié à, au moins deux électrodes cutanées (14).

11. Procédé selon la revendication 10, dans lequel l'étape de l'altération du champ électrique oscillant au moyen du dispositif de stimulation implantable (10) est réalisée au moyen de l'ouverture et de la fermeture en alternance d'un commutateur d'autoshort (62) ou du commutateur d'impulsion de stimulation (58 ; 58.1, 58.2, 58.3, 58.4), respectivement, au cours d'une période d'autoshort suivant la délivrance d'une impulsion de stimulation afin de moduler une impulsion d'autoshort ou une amplitude du pic d'une impulsion de stimulation, respectivement.

12. Procédé selon au moins une des revendications 10 ou 11, dans lequel l'étape de la détection du changement d'un champ électrique local comprend la détection d'un courant ou d'une tension par les électrodes cutanées (14) et l'intégration d'une tension ou d'un courant détecté par un nombre de cycles du champ électrique oscillant, ledit nombre de cycles correspondant à un facteur de division de la fréquence d'un diviseur de fréquence (20) du dispositif implantable (10).
